# EUROPEAN PATENT SPECIFICATION

(11) **EP 0 338 589 B1**
(45) Date of publication and mention of the grant of the patent: **25.09.1996**
(21) Application number: 89107264.7
(22) Date of filing: 21.04.1989
(51) Int. Cl.: A61J 1/00, A61K 33/18, A61F 6/00

(54) **Microbicidal cleanser/barrier kit for preventing the transmission of aids and other sexually transmitted diseases**
Mikrobentötende Reinigungsmittel-, Bariere-Ausrüstung zur Verhütung der Übertragung von AIDS und von anderen sexuell übertragbaren Krankheiten
Nettoyant et barrière microbicide pour empêcher la transmission du SIDA et d'autres maladies sexuellement transmissibles

(30) Priority: 22.04.1988 US 184842
(43) Date of publication of application: 25.10.1989
(73) Proprietor: Stoner, Fred Leroy, Las Vegas Nevada 89117 (US)
(72) Inventor: Stoner, Fred Leroy, Las Vegas Nevada 89117 (US)
(74) Representative: Baillie, Iain Cameron

(56) References cited:
- WO-A-81/00207
- DE-A- 3 605 361
- US-A- 2 391 094
- US-A- 2 409 952
- US-A- 2 538 197
- US-A- 3 184 121
- US-A- 3 245 525
- US-A- 4 301 916
- US-A- 4 344 535
- US-A- 4 466 956
- MORBIDITY AND MORTALITIY WEEKLY REPORT, Vol. 37, no9, 11th March 1988, pages 133-137; US. DEPARTMENT OF HEALTH AND HUMAN SERVICES: "Condoms for prevention of sexually transmitted diseases"

## Description

### Field of the Invention

This invention relates to combinations of drugs, germicides, cleansers, and microbicidal barriers to prevent the transmission of sexually transmitted diseases using a combination of these articles, more specifically to a a prophylactic kit for use in connection with sexual intercourse for preventing the transmission of Acquired Immune Deficiency Syndrome (AIDS) plus other sexually transmitted diseases.

### Background of the Invention

Sexually transmitted diseases (STD) can be defined as diseases that can be transmitted from one person to another primarily during sexual intercourse activities and body contacts between an infected individual and an uninfected individual. There are currently over twenty-six (26) recognized STD's including AIDS, Syphilis, Gonorrhea and Herpes. The contact sites believed to be the most responsible for STD transmission are the genitals, rectum, mouth and hands. Because of the variety of sexual and related contacts between these contact sites, no single treatment of a single site is known to be fully effective in preventing the transmission of STD's.

A combination of microbicidal agents, cleansing agents and barriers for preventing the transmission of sexually transmitted diseases should be easy to use, reliable pleasing to the senses and low in cost. More importantly, the materials and method must have a high effectiveness in preventing AIDS and other sexually transmitted diseases in real life sexual encounters, not only a laboratory environment.

Current devices and methods to prevent the transmission of AIDS and other venereal diseases tend to fall into three categories. The first is a solid barrier, such as a condom or diaphragm or vaginal sponge, which attempts to prevent a portion of one partner's skin and body fluids from contact with the other partner's skin or body fluids. The second method is a chemoprophylaxis or antibiotic injection or oral administration, which attempts to suppress the activity of the microbial agents that enter the body and cause the sexually transmitted diseases. The final method is the use of coating fluids (foams, creams, jellies) on affected portions, which form a viscous barrier to microbes and reduce abrasion. Some combinations of these three basic methods are known, as well as in combination with other treatment objectives, for example, birth control foams combined with spermicide compounds, such as nonoxynol-9, that also act as anti-microbial agents.

These prior approaches have many limitations, primarily related to the uncontrolled variety of body and fluid contact sites before, during and after sexual activity, and the multiplicity of microbial agents involved. Although the first (barrier) method, if used properly, can significantly reduce the incidence of sexually transmitted diseases, it may increase irritation, allergic reactions, and introduce additional microbes. Since it is believed that at least some of the sexually transmitted diseases can only be transmitted through a break in the skin, these side effects might actually cause transmission of a disease which would otherwise not be transmitted.

Other limitations are also that the barrier does not protect uncovered portions at or near the genitals, such as the testicles or rectal area, or non-genital areas, such as the mouth and hands. These uncovered portions are also frequently in contact with the sexual partner or fluids from the sexual partner. In addition, placement and especially removal of a contaminated barrier can also transmit disease. Solid barriers have also been used in combination with anti-microbial agents and coating fluids. Although improved protection resulted, the microbial agent caused added irritations, allergies and candidiasis infections in many of users. In addition, this combined method still did not address some of the limitations previously noted, such as before and after contacts with exposed/contaminated but unprotected including: the mouth; hands; and paragenital areas.

Studies conducted on the effectiveness of various antimicrobial agents placed in the genital areas (the second method) in preventing sexually transmitted diseases, including triethanolanime in oil, mapharsen and sodium laurel have shown a decrease in the rates of some sexually transmitted diseases, but increases in the rates of non-gonoccal urethritis, candidiasis, anti-microbial resistances, and allergic reactions. Vaginal drying has also been noted in other studies. It was recognized that finding an antibiotic that can be totally effective against the variety of microbial agents involved in sexually transmitted diseases, 1) without side effects of repeated use and 2) in the variety of human contacts involved in sexual encounters would be unlikely, if not impossible, and recent research has concentrated in other areas. Many of the limitations previously discussed were also present, such as the protection of non-genital surfaces.

Tests of the third method using a coating fluid, especially in conjunction with a spermicide with antimicrobial properties as well, have again shown decreased incidence in some sexually transmitted diseases, but significant side effects (irritations, allergies, vaginal drying, and infections) were again observed.

None of the prior art known to the applicant incorporates treatment and protection of all the paragenital and non-genital areas that may be involved during a sexual encounter. None of the prior art known to the applicant uses mild, previously non-specific anti-microbial agent with no known side effects and derived from a natural element, either alone or in conjunction with barriers and/or viscous fluids. None of the prior art known to the applicant provides a combination of articles to be applied to all of the paragenital and non-genital areas to prevent the transmission of STD's. Although rinsing and douche of the genitals is known, none of the prior methods for preventing sexually transmitted diseases known to the applicant incorporation a douche and/or cleansing of all the affected areas. Although other antiseptic mouthwashes have been long known and used for therapeutic purposes, no prior method known to the application uses a mouthwash for preventing sexually transmitted disease.

U.S. Patent No. 4,301,916 (Handelman) describes a carrying case for contraceptive devices. The case includes a series of pouches and a compartment which holds various elements used in a birth control system. The pouches have an entrance side which has elastic closing means for holding partially enclosed devices in place. A hinged lid is also provided. The compartment fully encloses an additional device in manners designed to permit easy cleaning.

U.S. Patent No. 4,466,956 (Leeds) discloses a method of therapy for oral herpes simplex. The method comprises the serial application of povidone-iodine, and, immediately thereafter, the application of a corticosteroid, preferably triamcinolone acetonide. The latter is used as an anti-inflammatory agent and adhesive. The serial treatment is repeated once or twice a day for several days.

WO 81/00207 (Harrigan) describes a pharmaceutical preparation which is a topical, composition for rendering the skin aseptic. The preparation consists of a combined form of iodine and an agent for promoting the absorption and dispersal of the composition in the epidermis. In a preferred embodiment disclosed in this application, the iodine is elemental iodine in polyvinylpyrrolidone.

In view of the rapid spread of the AIDS epidemic, what is urgently needed is a simple combination of devices and a method of prevention using these devices which does not require the use of costly pharmacopoeia and does not interfere with the enjoyment of the sexual encounter. It would also be beneficial to present these devices in a convenient package grouping all the elements necessary to quickly and effectively practice the preventive method.

### Summary of the Invention

According to one aspect of the invention there is provided a prophylactic kit for use in connection with sexual intercourse and related intimate contact between partners, which comprises a sealed enclosure, and therein a condom; characterized by a first container holding a quantity of a first liquid solution of povidone-iodine, the first solution containing between 2%-12% by weight of povidone-iodine; a second container holding a quantity of a second liquid solution of a germicidal agent, the second liquid solution containing between 0.25% and 5% by weight of povidone-iodine; wherein said first solution is formulated for topical applications, and said second solutions is formulated for use as a mouthwash; at least one solution applicator; and written indications and instructions for use of the kit before and after said sexual intercourse and related intimate contact.

The invention nay provide a safe and effective application of a combination of cleansing agent, microbial agent and barrier in a method applying these devices to prevent the transmission of sexually transmitted diseases, including AIDS.

The invention nay also essentially eliminate significant side effects from prevention method and associated combination of devices.

The kit combines a condom, at least one cleaning implement, and a plurality of coating and antimicrobicidal solutions containing povidone-iodine complex. The solutions include a before-contact mouth solution, a before-contact body solution, an after-contact mouth solution, and an after-contact body solution. Up to three hours before sexual contact, the solution is applied over the exposed areas of skin and areas are cleansed with the before contact solutions and cleaning implements, such as a toothbrush for cleaning the mouth. This includes hands, mouth, genitals, lower abdomen, upper thighs and rectum. The solutions also produce a coating action that functions as a sustained chemical barrier and a coating which covers minor cuts or abraded areas before sexual contact. During sexual contact, the coatings also act as a lubricant, reducing further abrasion. After sexual contact, the after contact solutions and cleaning implements are used to cleanse the exposed or potentially contaminated areas and the condom is removed. The solutions further reduce microbial activity and once again coat and heal any abraded areas. The mouth solutions may be in the form of toothpaste and or gargle and contain between 0.25 percent by weight and 5 % by weight of povidone-iodine with the remainder primarily water. Both types of solutions are non-staining, water soluble and can be pleasantly flavored and scented for ease of use and sensory pleasure. Cleaning implements can include toothbrush, wash cloths and brushes. In the preferred embodiment of the invention the kit is boxed in a cigarette-pack sized box for convenient dispensing from cigarette or candy vending machines.

### Brief Description of the Drawings

FIGURE 1 shows a perspective view of a single use prevention kit enclosure.

FIGURE 2 shows a side view thereof with open cover.

FIGURE 3 shows a front view thereof.

FIGURE 4 shows an exploded view of a single use prevention kit enclosure and contents.

FIGURE 5 shows a front cross sectional view thereof.

FIGURE 6 shows a left side cross sectional view thereof.

FIGURE 7 shows a right side cross sectional view thereof.

FIGURE 8 shows a top cross sectional view thereof.

FIGURE 9 shows a bottom cross sectional view thereof.

FIGURE 10 shows an exploded side perspective view of a single use prevention kit enclosure and contents.

FIGURE 11 shows an exploded side view perspective view of a multiple use pack.

FIGURE 12 shows a block diagram of a preventive method.

### Description of the Preferred Embodiment

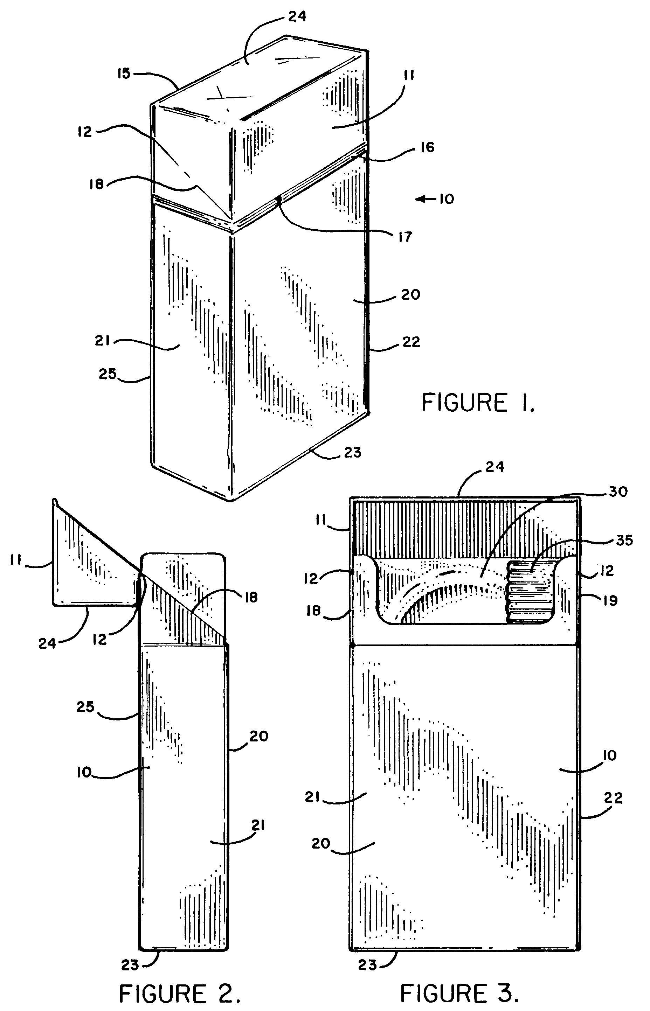

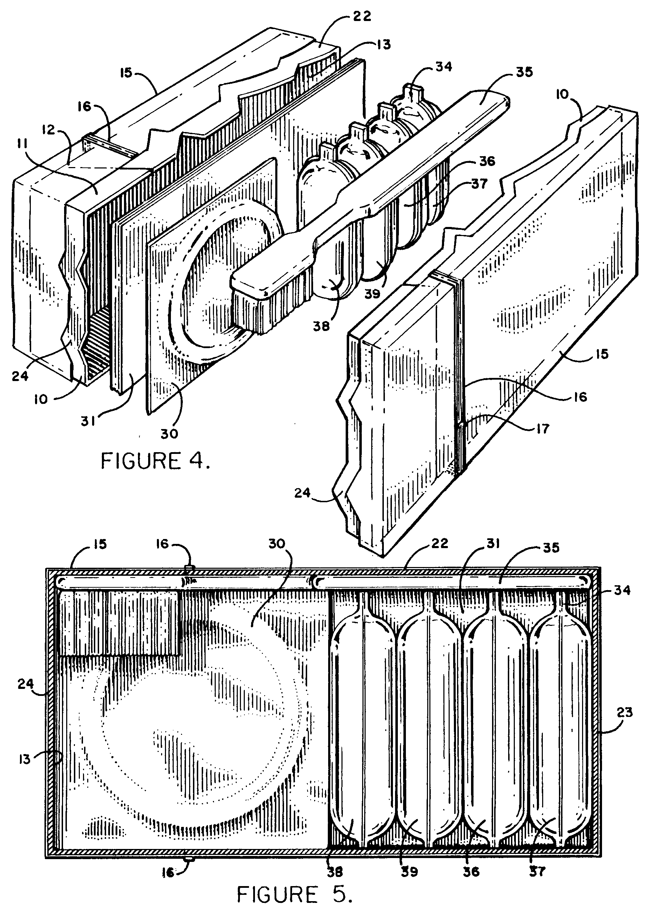

FIGURE 1 shows a side perspective view of a single use prevention kit enclosure. A cigarette type of flip up box 10 is used to package the elements of the preferred embodiment of the prevention kit. The box 10 is composed of a lid 11, a lid joint or box handle 12, an interior space in the box for storing the elements of the kit (item 13 shown in Figure 4), a transparent foil or cellophane seal or wrapping 15, a tab strip 16 for opening of the cellophane 15 of box 10, a tab or means for beginning to opening 16 the cellophane 15, box edges matching lid 11 (left matching edge 18 and right matching 19 shown in Figure 3), a front of box 20, a left side 21 of box 20, a right side 22 of box 20, a bottom 23 of box 20, a top 24 of box lid 11, and a back 25 of box 20 (shown in Figure 2). The overall size, shape and configuration is selected to fit into cigarette vending machines or similar dispensing apparatus.

FIGURE 2 shows a left side view of a single use prevention kit box 10 having the lid 11 open. The lid 11 and top 24 folds on joint 12 to back 25. Left side matching edge 18 is now exposed along with the elements contained in the box 10. The front 20, bottom 23 and left side 21 form the remainder of the box 10 visible from this view.

FIGURE 3 shows a front view of a single use prevention kit box 10 having the lid 11 open exposing the contents or elements of the prevention kit. The elements consist of condom package 30, a folded instruction sheet 31 (see Figure 4), a tooth brush 35, and four containers of solutions (see Figure 4) including tear off tops (34) of a before-contact oral solution (36), an after-contact oral solution (37), a before-contact body solution (38), and an after-contact body solution (39). These elements are protected and contained by lid top 24, lid 11 and lid hinge 12, left side matching edge 18, right side matching edge 19, left side 21, right side 22, front 20 and bottom 23.

FIGURE 4 shows an exploded view of a single use prevention kit. Box 10 encloses a single condom package 30, sheet of instructions 31, single use containers having tear off tops 34 of before-contact oral solution 36, after-contact oral solution 37, before-contact body solution 38 and after-contact body solution 39. Access to the contents of the box is provided by a tab 17 opening the tab strip 16, allow one to open lid 11 and top 24 along hinge 12. The interior 13 of box 10 is formed by the right side 22 and remained side, back, front and bottom of box 10.

FIGURE 5 shows a front cross sectional view of a single use prevention kit lying on one side. The four single use containers, 36 - 39 are composed of half shell plastic moldings joined to have ported neck 34. Breaking the neck seal of these single use disposable containers allows the generally liquid solution to be poured or squeezed out of the containers. Containers would be individually labeled and/or color coded. In preferred embodiment, the containers are of equal size. The same solution fills the before-contact oral and after-contact oral containers. Similarly, another solution fills both the before-contact body and after-contact body containers. However, other embodiments could have unequal sized containers and various solutions of povidone-iodine. The preferred embodiment places the containers on one side of the condom package and toothbrush, but alternate placements are also possible. The contents are again contained in the interior 13 by bottom 23, right side 22, top 24, cellophane 15 and tab strip 16.

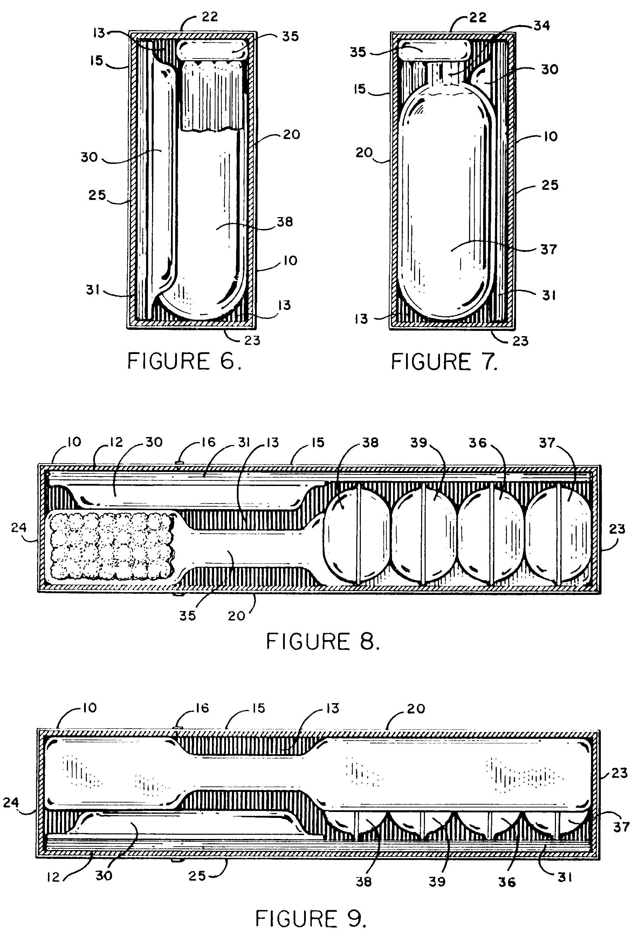

FIGURE 6 shows a left side cross sectional view of a single use prevention kit lying on its sides as viewed from the left side of the view shown in Figure 5. The edge view of the instruction sheet 31 is adjacent to condom package 30, which is adjacent to the toothbrush 35, which partially obscures one of the containers 38 (other containers not visible in this view). The contents of the interior 13 of box 10 are again contained by front 20, bottom 23, right side 22, back 25 and cellophane 15.

FIGURE 7 shows a right side cross sectional view of a single use prevention kit. A full side view of one of the containers 37 adjacent to the sheet of instructions 31 partially obscures the toothbrush 35 and condom package 30 in box 10. The contents of the interior 13 of box 10 are again contained by front 20, bottom 23, right side 22, back 25, and cellophane 15.

FIGURE 8 shows a bottom cross sectional view of a single use prevention kit. The sheet of instructions again lies adjacent to condom package 30, which lies adjacent to toothbrush 35 which is partially obscured by the four containers 36 - 39 in the interior 13 of box 10. The contents of the interior 13 of box 10 are again contained by front 20, bottom 23, left side 21, top 24 joined at hinge 12, and cellophane 15, tab strip 16.

FIGURE 9 shows a top cross sectional view of a single use prevention kit. The sheet of instructions again lies adjacent to condom package 30, which lies adjacent to toothbrush 35 which is partially obscured by the four containers 36 - 39 in the interior 13 of box 10. The contents of the interior 13 of box 10 are again contained by front 20, bottom 23, right side 20, left side 21, top 24 joined at hinge 12, and cellophane 15, tab strip 16.

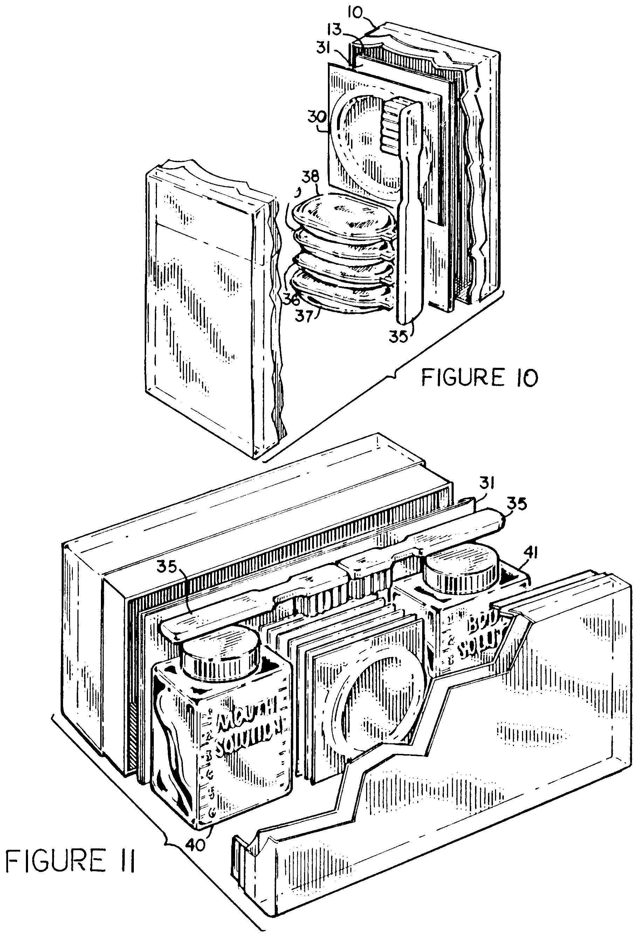

FIGURE 10 shows an exploded side perspective view of a single use prevention kit. The interior 13 of box 10 may be a separate padded insert to protect the contents and devices contained in the kit. The devices are a disposable toothbrush 35, two disposable containers of oral solution 36 and 37, two disposable containers of body solution 38 and 39, and a sheet of instructions 31.

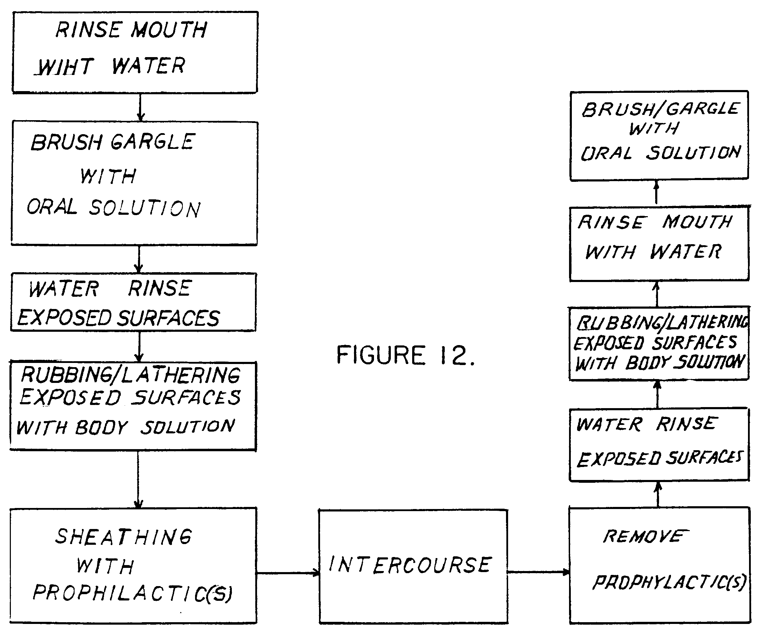

FIGURE 11 shows an exploded side view perspective view of a multiple use pack. Multiple toothbrushes 35 are provided with multiple condom packages. The oral solution in container 40 is a mouth wash and body solution in container 41 is a liquid soap. Multiple use containers hold sufficient amounts for multiple applications of the solutions. The sheet of instructions 31 would contain the specific amounts of solutions to be used for before- and after-contact uses, such as a capful. Multiple use packages could be provided for a single use by both sexual partners or a plurality of uses by a single user.

FIGURE 12 shows a block diagram of a preventive method of using the preferred embodiment. The initial step is to rinse the mouth with water, followed by brushing with a portion of the before contact oral solution, followed by a gargle of the remaining solution used as a mouth wash. An alternative would be to package the before contact solution in two packages, one having the consistency of a toothpaste, the other having the consistency of a mouth wash. The brushing and gargling can be followed by a second rinse with water. The first rinse, brushing, gargling and second rinse cleanses the mouth of foods and residue that may act as a growth media for STD microbes and coats the mouth acting as a broad, nonspecific anti-microbial agent plus a coating action over minor cuts or sores. An alternate, but less effective method is to delete the initial water rinse, relying upon brushing or gargling with the oral solution to cleanse as well as the germicidal and coating actions.

Although research indicates this solution kills the HIV virus (AIDS causing microbe) on contact, three minutes of the oral solution in contact with the mouth is recommended in order to distribute the solution and accomplish a broad spectrum of microbicidal activity. The solution may be combined with a flavoring to make a more palatable toothpaste and/or mouth wash. The active ingredient may also be added to toothpaste, gargle, or breath spray in alternate applications. The active ingredient in the first solution is povidone-iodine, a complex obtained by reacting iodine with the polymer polyvinylpyrrolidone in an oral solution of at least 0.25 percent by weight of povidone-iodine. The active ingredient may be 1-Ethenyl-2pryrrolidinine homopolymer compound with iodine or 1-vinyl-2-pyrrolidone polymers, iodine complex. Concentrations of up to 5.0 percent by weight in the oral solution are also expected to be effective without serious side effects. The remainder of the solution is primarily water, but may also include a buffering compound such as sodium bicarbonate. This solution has been long used for the treatment of sore throats and inflammations of the mouth, but not as a preventive treatment for sexually transmitted diseases. Prior use has shown it to be non-irritating with essentially no significant side effects.

A before-contact body solution is applied to potential exposed skin contact areas, including genitals, up to three (3) hours before sexual contact, including hands, face, lower abdomen, upper thighs and rectum. The preferred embodiment incorporates a water rinse prior to application of the body solution, but an alternate embodiment could use a single application, having the water and/or detergent in the solution serve the entire cleansing purpose. The before-contact body solution can be in the form of a liquid or solid soap, applied while in a shower or while standing in a bath tub. It may also be impregnated into a disposable wash cloth or brush for application. The solution may also be in the form of a gel, aerosol, bath or body oil, to be applied separately or after a rinse or bath. Rinsing with water before and after application of the before contact body solution assists cleaning action, but is not necessary. Even after rinsing, a residue is left on the skin that is resistant to rubbing off (including rubbing off on clothing) and provides up to three (3) hours of protection. The method using the preferred embodiment is to rinse the genital area, rectum, lower abdomen, upper inner thighs and around the mouth with water. The sealed port of the single use container of before contact body solution is manually opened and the liquid contents are poured into the hands, worked into a lather and applied to the genital area, rectal area, lower abdomen, upper inner thighs and around the mouth. The lather is allowed to remain on the body for three (3) minutes to assure full effectiveness. The lathered areas may then be rinsed with water.

The before-contact body solution is a more concentrated solution of povidone-iodine in a sudsing and coating liquid, such as a detergent. It essentially retains the broad microbicidal spectrum of iodine, yet virtually without undesirable side effects. Again, it has long been used for skin cleansing, including as a douche, to degerm topical skin areas of patients and to help prevent skin infections, but not as part of a sexually transmitted disease prevention method. The skin is wetted with water and sufficient solution added to work up a lather. Although research shows the before contact body solution kills the HIV virus on contact, retaining the solution in contact with the skin for approximately three (3) minutes is recommended to achieve the broad spectrum of microbicidal results. After allowing the lather to remain for approximately these few minutes to optimize microbicidal properties, lather may be rinsed. However, even after rinsing, a major portion of the active ingredient remains on the skin and genital areas.

The body solution is recommended to contain between 7.5 and 10 percent by weight of povidone-iodine, but is also expected to be generally effective in a range of between 2.0 and 12 percent by weight without serious side effects. The remainder of the body solution is primarily water, but again may also contain sodium bicarbonate or other similar compound to control the pH of the solution. An alternate embodiment would have the povidone-iodine complex dissolved in primarily in alcohol solution. The body solution is also expected to be somewhat effective in a concentration as low as 0.25 percent by weight. Although the more concentrated solutions may stain starched linen or clothes, the stain can be washed with soap and water. The solution may also contain flavorings and/or scents, if desired for sensory stimulation. In alternate embodiments, the active ingredient may also be added to soap, perfume, toilet water, wash sponges or cloths, bubble bath, bath oil, bath powder, douche, enemas, towels or towelettes, creams, spermicidal birth control compounds, diaphragms, creams, jellies, aerosols, vaginal sponges and caps, and lotions. These other products that can come in contact with the exposed skin or orifice areas would contain the added active ingredient of povidone-iodine and may be used in addition to the first and second solutions.

The condom is individually packaged to maintain sterile conditions and is applied to the penis after the hands and penis are cleaned and coated with the before contact solution. If the hands and/or penis have been exposed to one of the sexual partner's body fluids after application of the solutions but before application of the condom, additional before-contact solution or after-contact solution (equal in the preferred embodiment) should be applied to the affected areas. The condom should be placed/rolled onto an erect body solution treated penis. The application of the body solution is not expected to affect the birth control effects of the condom and the method may be applied for both purposes.

In pre-intercourse and intercourse activities, fluid exchanges and skin contacts may occur, but all contact areas now have been treated and coated. The preferred embodiment combines the pretreatment cleaning and coating just described, with the proper application and use of a solid barrier as well, such as a condom. Sexual contact may also include the added or combined use of birth control compounds, such as spermicidal creams or other lubricants, vaginal sponges or other implements.

An alternate embodiment comprises the additional solutions and treatments with povidone-iodine of other products and implements that come in contact with exposed skin or orifice areas before or after sexual contact and exposure. This may require changes to the concentration of the two (oral and body) solutions. Concentration of the active ingredient would be similar to the ranges previously described, but tend to be less concentrated to avoid serious side effects in areas receiving multiple applications of the active ingredient.

After sexual contact, the condom is carefully removed so the penis does not come into contact with partner's fluids that is present on the condom's exterior, or may be on the testicles, lower abdomen and upper thighs. Careful inspection of the condom for tears and/or leaks is also recommended.

After sexual contact and careful removal of the solid barrier, the after-contact body solution container is opened and liquid is applied as soon as possible after sexual contact. The after-contact application is recommended to be immediately following the sexual contact, but beneficial effect may be achieved up to six hours after the first sexual contact. The genital areas, rectum, lower abdomen, upper thighs and around the mouth are rinsed with water. The individually packaged after contact body solution is opened and applied to the wet hands and skin, lathered and preferably left standing for three minutes, and rinsed. The same or different flavorings and/or scents may again be added to the solution. The solution destroys microbes on contact, medicates exposed skin areas and also tends to heal or at least coat any new or existing minor irritations and abrasions.

The final process step is to rinse and clean the mouth as soon as possible after sexual exposure with the after contact oral solution, or similar solution. However, beneficial effect may be achieved by rinsing up to three (3) hours after sexual exposure. Again, the same or different flavoring may be added for sensory stimulation. The form may again be a mouth wash, toothpaste, gargle/rinse in one or more packages. In the preferred embodiment, half the contents of the individually packaged after-contact oral solution is applied to the disposable brush and the gums and teeth are brushed. The remaining oral solution is gargled and allowed to remain for three (3) minutes. The gargle/toothpaste is then rinsed. This process cleanses the mouth and throat, kills microbes and provides a coating action against further contamination especially of irritated and abraded areas.

Alternate process steps could comprise applying the before-contact body solution prior to the before-contact oral solution and after-contact oral solution before the after-contact body solution.

An alternate method using the active ingredient without the before-contact and after-contact applications of the solutions would be to include or apply the povidone-iodine directly to vaginal sponges, condoms, contraceptive foams and jellies, contraceptive creams, aerosols, toilet articles previously described (soap, perfume, toilet water, wash sponges or cloths, bubble bath, bath oil, bath powder, douche, enemas, towels or towelettes, and lotions) or combined with other products that may contact exposed skin or orifice areas. These products may not serve to cleanse the genital surfaces, but may provide some microbicidal and barrier protection. These other products incorporating povidone-iodine could be used separately and without multiple applications and cleansing solutions. Concentration ranges of the povidone-iodine would be altered from a minimum in these other applications to achieve minimum acceptable microbicidal and barrier properties, to a maximum where no significant adverse side effects were observed in these applications.

It is expected that the preferred embodiment cleaning implements, solutions and method described will be effective against at least 98% of microbes and viruses that are responsible for sexually transmitted disease, including AIDS. The cleaning and sustained microbicidal and viricidal barrier and coating of minor cuts/abrasions is expected to last for three (3) hours, which appears to be especially important in this application. The cleansing action also eliminates possible environmental contributory factors from the skin and orifices (surface bacteria, fungus, yeast, skin oil, perspiration, etc.).

The invention is now described with reference to an example.

### EXAMPLE

The effect of the preferred embodiment on the causative microbes of a number of sexually transmitted diseases were researched or tested in the sexual encounter application as previously described. Sexual encounter was/or was expected to be within three hours of the first and second applications of the solutions. The solutions were or were assumed to be in contact for a minimum of approximately two (oral) or three (skin) minutes prior to discharge or rinsing. The solutions used or analyzed were essentially as previously described. Table 1 lists the results of the research/testing.

**Table 1**

| Disease | Causative Microbes | Microbicidal Effect |
|---|---|---|
| Coliforms | Escherichia coli, Aeromonas, Enterobacters, Campylobacters, Shigella, Proteus Salmonella, and Pseudomonas | positive |
| AIDS | H I V | positive |
| Herpes | Herpes Simplex I & II | positive |
| Cytomegalovirus | Cytomegalo virus | positive |
| Syphilis | Treponema Pallidum | positive |
| Tricomonas | Trichomonas vaginalis | positive |
| Entamoeba | Entamoeba histolytica | positive |
| Mycoplasma | Mycoplasma hominis | positive |
| Gradnerelia | Gardnerelia vaginalis | positive |
| Cornynevacterium vaginalis | Gardnerelia vaginalis | positive |
| Haemophilis vaginalis | Gardnerelis vaginalis | positive |
| Ureaplasma | Ureaplasma urealyticum | positive |
| Chlymedia | Chlymedia trachomatis | positive |
| Gonorrhea | Neisseria gonorrhea | positive |
| Candidiasis | Candida albicans | positive |
| Strept. infections | Streptococcus faecalis | positive |
| Staph. infections | Staphyloccus aureus | positive |
| Group A & B | Streptococcus -beta hemolytic (group A & B) | positive |
| Lymphogranuloma venereum | Chlymedia trachomatis | positive |

While the preferred embodiment and alternate embodiments of the invention have been shown and described, changes and modifications may be made therein within the scope of the appended claims, without departing from the scope of this invention.

## Claims

1. A prophylactic kit for use in connection with sexual intercourse and related intimate contact between partners, which comprises:
a sealed enclosure (10), and therein:
a condom (30);
characterized by :
a first container (36) holding a quantity of a first liquid solution of povidone-iodine, the first solution containing between 2%-12% by weight of povidone-iodine;
a second container (37) holding a quantity of a second liquid solution of a germicidal agent, the second liquid solution containing between 0.25% and 5% by weight of povidone-iodine;
wherein said first solution is formulated for topical applications, and said second solution is formulated for use as a mouthwash;
at least one solution applicator; and
written indications and instructions (31) for use of the kit before and after said sexual intercourse and related intimate contact.

2. The kit as claimed in Claim 1 characterized by:
a third container (38) holding a quantity of said first solution; and
a fourth container (39) holding a quantity of said second solution;
wherein first (36) and second (37) containers are marked for use before sexual intercourse; and
said third (38) and fourth (39) containers are marked for use after sexual intercourse.

3. The kit as claimed in Claim 2 further characterized by at least one toothbrush (35) within said sealed enclosure (10).

4. The kit as claimed in Claim 2 characterized in that the first solution contains a detergent.

5. The kit as claimed in Claim 2 characterized in that the solutions are in a generally liquid form.

6. The kit as claimed in Claim 1 wherein the contents thereof are packaged in container having dimensions approximating that of a cigarette pack.

## Patentansprüche

1. Prophylaxeeinheit zur Verwendung im Zusammenhang mit Geschlechtsverkehr und dem damit verbundenen intimen Kontakt zwischen Partnern, umfassend:
ein verschlossenes Gehäuse (10), und darin:
ein Kondom (30);
gekennzeichnet durch:
einen ersten Behälter (36) mit einer Menge einer ersten flüssigen Lösung von Polyvidon-Iod, wobei die erste Lösung zwischen 2 und 12 Gew.-% Polyvidon-Iod enthält;
einen zweiten Behälter (37) mit einer Menge einer zweiten flüssigen Lösung eines keimtötenden Mittels, wobei die zweite flüssige Lösung zwischen 0,25 und 5 Gew.-% Polyvidon-Iod enthält;
wobei die erste Lösung zur äußeren Anwendung formuliert ist, und die zweite Lösung zur Verwendung als Mundwasser formuliert ist;
mindestens einen Applikator zum Auftragen der Lösung; und
schriftliche Hinweise und Anweisungen (31) für die Verwendung der Einheit vor und nach dem Geschlechtsverkehr und dem damit verbundenen intimen Kontakt.

2. Einheit nach Anspruch 1, gekennzeichnet durch:
einen dritten Behälter (38) mit einer Menge der ersten Lösung; und
einen vierten Behälter (39) mit einer Menge der zweiten Lösung;
wobei der erste (36) und der zweite (37) Behälter zur Verwendung vor dem Geschlechtsverkehr gekennzeichnet sind; und
der dritte (38) und der vierte (39) Behälter zur Verwendung nach dem Geschlechtsverkehr gekennzeichnet sind.

3. Einheit nach Anspruch 2, des weiteren gekennzeichnet durch mindestens eine Zahnbürste (35) in dem verschlossenen Gehäuse (10).

4. Einheit nach Anspruch 2, dadurch gekennzeichnet, daß die erste Lösung ein Reinigungsmittel enthält.

5. Einheit nach Anspruch 2, dadurch gekennzeichnet, daß die Lösungen im allgemeinen flüssig sind.

6. Einheit nach Anspruch 1, deren Inhalt in einem Behälter verpackt ist, dessen Abmessungen ungefähr denen einer Zigarettenschachtel entsprechen.

## Revendications

1. Trousse prophylactique pour utilisation dans une relation sexuelle et un contact intime associé entre des partenaires, qui comprend :
une boîte fermée (10) comprenant un préservatif (30) ;
caractérisée en ce qu'elle comprend en outre :
un premier récipient (36) contenant une quantité d'une première solution liquide de povidone-iodine, la première solution ayant entre 2 et 12 % en poids de povidone-iodine ;
un deuxième récipient (37) contenant une quantité d'une deuxième solution liquide d'un agent germinicide, la deuxième solution liquide ayant entre 0,25 et 5 % en poids de povidone-iodine ;
dans lesquels ladite première solution est formulée pour des applications locales et ladite deuxième solution est formulée en tant que rince bouche ;
au moins un applicateur de solution ; et des instructions et des indications écrites (31) pour l'utilisation de la trousse avant et après la relation sexuelle et le contact intime associé.

2. Trousse selon la revendication 1, caractérisée par :
un troisième récipient (38) contenant une quantité de ladite première solution ; et
un quatrième récipient (39) contenant une quantité de ladite deuxième solution ;
dans laquelle le premier (36) et deuxième (37) récipients sont marqués pour utilisation avant la relation sexuelle ; et
lesdits troisième (38) et quatrième (39) récipients sont marqués pour utilisation après la relation sexuelle.

3. Trousse selon la revendication 2, caractérisée en outre par au moins une brosse à dents (37) disposée dans la boîte fermée (10).

4. Trousse selon la revendication 2, caractérisée en ce que la première solution contient un détergent.

5. Trousse selon la revendication 2, caractérisée en ce que les solutions sont généralement sous forme liquide.

6. Trousse selon la revendication 1, dans lequel les contenus sont emballés dans un contenant présentant des dimensions approchant celles d'un paquet de cigarette.
